# EUROPÄISCHE PATENTSCHRIFT

(11) **EP 0 837 659 B1**
(45) Veröffentlichungstag und Bekanntmachung des Hinweises auf die Patenterteilung: **24.11.1999**
(21) Anmeldenummer: 96921873.4
(22) Anmeldetag: 01.07.1996
(51) Int. Cl.: A61C 13/00

(54) **VERFAHREN UND VORRICHTUNG ZUR RECHNERGESTÜTZTEN RESTAURATION VON ZÄHNEN**
PROCESS AND DEVICE FOR COMPUTER-ASSISTED RESTORATION OF TEETH
PROCEDE ET DISPOSITIF DE RESTAURATION DENTAIRE ASSISTEE PAR ORDINATEUR

(30) Priorität: 07.07.1995 DE 19524855
(43) Veröffentlichungstag der Anmeldung: 29.04.1998
(73) Patentinhaber: Sirona Dental Systems GmbH, 64625 Bensheim (DE)
(72) Erfinder: FRANETZKI, Manfred, D-64625 Bensheim (DE)
(74) Vertreter: Isenbruck, Günter, Dr.
(86) Internationale Anmeldenummer: DE9601166
(87) Internationale Veröffentlichungsnummer: WO9702788

(56) Entgegenhaltungen:
- EP-A- 0 373 077
- US-A- 4 575 805

## Beschreibung

In der US-PS 4 575 805 ist ein Verfahren und eine danach arbeitende Vorrichtung zur rechnergestützten Restauration von Zähnen beschrieben, bei dem (der) in einem ersten Schritt zunächst die Geometrie des zu restaurierenden Zahnes und eventuell seiner Umgebung, d.h. auch der Gegenzähne, mit einer vorzugsweise optischen Meßvorrichtung aufgenommen und die erhaltenen Werte in einem Speicher abgelegt werden. Als Meßeinrichtung wird dort eine 3D-Meßkamera verwendet, mit der von dem aufzunehmenden Objekt, dem präparierten Zahn, ein monochromes 3D-Bild erzeugt wird, indem auf das Objekt eine opake, diffus reflektierende Schicht aufgebracht wird und anschließend das Objekt mit einer monochromatischen Lichtquelle beleuchtet wird.

In einem zweiten Schritt wird das Bild vom Anwender interpretiert, d.h. es müssen Bodenlinien, Kavitätenränder, Äquatorlinien, Höcker, etc. erkannt und in das Bild eingezeichnet werden. Dies geschieht größtenteils von Hand durch den erfahrenen Arzt. In einem dritten Schritt wird das Restaurat (Inlay, Onlay, Krone, Veneer etc.) konstruiert. Diese Aufgabe erfordert professionelle CAD-Arbeit vom Bediener mit dreidimensionalem Vorstellungsvermögen und der Fähigkeit, am Bildschirm mit Computermitteln zu konstruieren.

Die Qualität des Restaurats hängt dabei wesentlich von diesen Fähigkeiten und vom Trainingszustand des Anwenders ab. In einem vierten Schritt wird das Ergebnis dieser Konstruktionstätigkeit in ein Programm für eine numerisch gesteuerte Schleif-/Fräsmaschine übersetzt. In einem fünften Schritt wird schließlich das Restaurat in der NC-Maschine aus einem Materialblock gefertigt. Im letzten Schritt wird das Restaurat in den Kiefer eingegliedert.

Ein Problem stellt die Bildinterpretation als Voraussetzung für eine gelungene Rekonstruktion dar. Bei dem heute üblichen Verfahren wird - wie eingangs bereits angesprochen -die betroffene Oberfläche vorher mit einem opaken, diffus reflektierenden Material beschichtet und der Gegenstand dann mit einer monochromatischen Lichtquelle beleuchtet. Das damit vom Gegenstand unter dieser Schicht erhaltene monochrome Bild erschwert die Identifikation des abgebildeten Materials. So kann nicht immer eindeutig erkannt werden, ob es sich bei dem abgebildeten Teil um einen Zahn oder um Zahnfleisch handelt, oder ob krankes und gesundes Material abgebildet wird.

Der im Anspruch 1 angegebenen Erfindung liegt die Aufgabe zugrunde, die 3D-Bildinterpretation zu verbessern.

Erfindungsgemäß wird vorgeschlagen, außer dem monochromatischen 3D-Meßbild auch Farbbilder aufzunehmen und diese für die Bildinterpretation und Restauration zu nutzen, indem man die Farbbilder mit dem 3D-Meßbild zur Überlagerung bringt, wobei die beiden Aufnahmen mit ein und derselben Kamera erstellt werden. Die 3D-Meßkamera ist so modifiziert, daß sie auch Farbbilder aufnehmen kann. Hierzu kann wie folgt vorgegangen werden:

Das Objekt wird nicht mit monochromatischem Licht, sondern mit weißem Licht beleuchtet. Die Kamera enthält anstelle von einem Schwarz-Weiß-CCD einen Farb-CCD mit einer vorgesetzten Farbfiltermaske, wobei die vorgesetzte Farbfiltermaske jeweils für die spezifische Farbe des im CCD verwendeten Pixels und für die Farbe des Meßsystems (häufig im IR-Bereich durchlässig) ist. Die Kameraoptik ist dabei farbkorrigiert, d.h. alle Linsen und Prismensysteme sind chromatisch korrigiert. Die Zufuhr des Lichts kann über einen Lichtleiter oder auch über eine externe Lichtquelle erfolgen.

Alternativ kann das Meßobjekt bei der Farbaufnahme nacheinander mit den charakteristischen Farben rot, grün, blau beleuchtet oder das vom Objekt reflektierte Licht sequentiell in den drei Farben zerlegt werden. Die Kameraoptik ist hierzu wieder farbkorrigiert (Achromat). Der CCD ist über den sichtbaren Bereich bis zum Meßbereich empfindlich. Das reflektierte Licht wird in der Kamera sequentiell empfangen. Die dabei nacheinander entstehenden drei Bilder werden auf elektronischem Wege zu Farbbildern verrechnet. Die Beleuchtung in den drei Farben kann durch in oder außerhalb der Kamera angeordnete Lichtquellen erfolgen, wobei in letzterem Falle ein geeigneter Lichtleiter vorhanden sein muß. Vorteilhaft kann es sein, drei in verschiedenen Wellenlängen emittierende Laserdioden zu verwenden; alternativ kann auch ein rotierendes Farbfilter vor einer konventionellen Glühlampe angebracht sein. Die Schwarz-Weiß-3D-Bilder und die Farbbilder werden vorteilhafterweise gleichzeitig auf einem Bildschirm übereinander oder nebeneinander dargestellt. Besonders vorteilhaft ist es, beide Bilder überlagert auf einem Bildschirm darzustellen. Damit läßt sich ein Optimum an Interpretierbarkeit des Bildes erzielen, insbesondere lassen sich Linien, Kanten, Übergänge usw. wesentlich besser erkennen.

Die Erfindung wird nachfolgend anhand von Ausführungsbeispielen beschrieben.

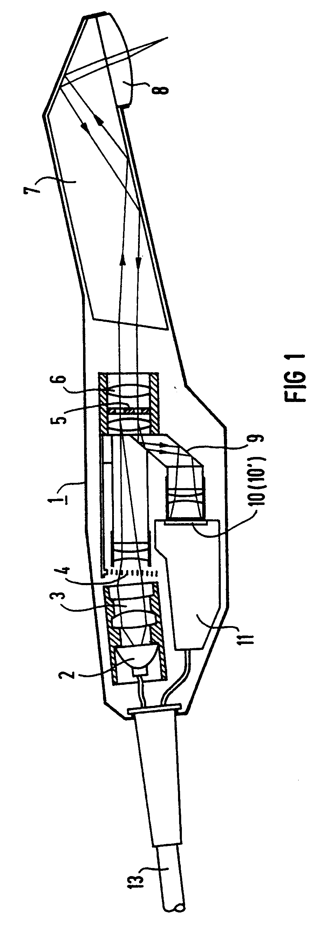

Die Figur 1 zeigt eine Ausführungsform einer herkömmlichen Meßkamera, wie sie in der eingangs genannten US-PS 4 575 805 offenbart ist. Eine solche, allgemein mit 1 bezeichnete Meßkamera enthält eine möglichst punktförmige monochromatische Lichtquelle 2, vorzugsweise eine LED, die im Infrarotbereich emittiert. Nach Durchgang durch einen Kondensor 3 wird das Licht auf ein oszillierendes Strichgitter 4 geworfen. Das Strichgitter 4 wird über das eine (obere) Loch einer Zweilochblende 5, ein Objektiv 6, ein Kopfprisma 7 und eine Feldlinse 8 auf das zu messende Objekt projiziert. Das reflektierte Licht gelangt auf umgekehrtem Wege über das andere (untere) Loch der Zweilochblende 5, ein Umlenkprisma 9 auf einen CCD-Sensor 10. Der CCD-Sensor ist hier ein Schwarz-Weiß-CCD-Sensor. In einer Bildelektronik 11 und einem nicht dargestellten, außerhalb der Kamera befindlichen Rechner werden die erhaltenen Bildinformationen zu 3D-Bildern verrechnet.

Es gibt nun zwei grundsätzliche Wege, die vorbeschriebene 3D-Meßkamera zu einer Farb-Videokamera zu modifizieren.

Das Objekt wird anstelle mit monochromatischem Licht mit weißem Licht beleuchtet und der Schwarz-Weiß-CCD-Sensor 10 durch einen Farb-CCD-Sensor 10' mit vorgesetzter Farbfiltermaske ersetzt. Die in der Kamera vorhandenen Linsen- und Prismensysteme sind nach üblichen optischen Verfahren chromatisch korrigiert (Achromaten). Die Lichtquelle kann dabei, wie in Fig. 1 gezeigt, in der Kamera oder auch extern in einem entfernten Lichtmodul angeordnet sein.

Als zweiter Weg wird alternativ vorgeschlagen, das Objekt nicht mit weißem Licht zu beleuchten und die Bilder mit einem Farb-CCD-Sensor aufzunehmen, sondern einen beliebigen, vorzugsweise einen Schwarz-Weiß-CCD-Sensor, vorzusehen und das Objekt sequentiell mit wenigstens drei Farben zu beleuchten, oder das vom Objekt reflektierte Licht sequentiell in die drei Farben zu zerlegen. Der Vorteil dieser Variante liegt unter anderem in einer wesentlich höheren Auflösung des CCD's (ein Pixel pro Bildpunkt, im Gegensatz zu drei Pixeln pro Bildpunkt bei einem Farb-CCD).

Die Beleuchtung mit drei verschiedenen Farben kann, wie beschrieben, durch die Kameraoptik, über getrennte Lichtleiter, die an der Kameraoptik vorbeigeführt werden, oder auch mit Hilfe einer externen Lichtquelle erfolgen.

Die drei Farben lassen sich z.B. durch drei gepulste LED's oder auch durch drei rotierende oder oszillierende Farbfilterscheiben vor einer üblichen Glühbirne oder Bogenlampe erzeugen.

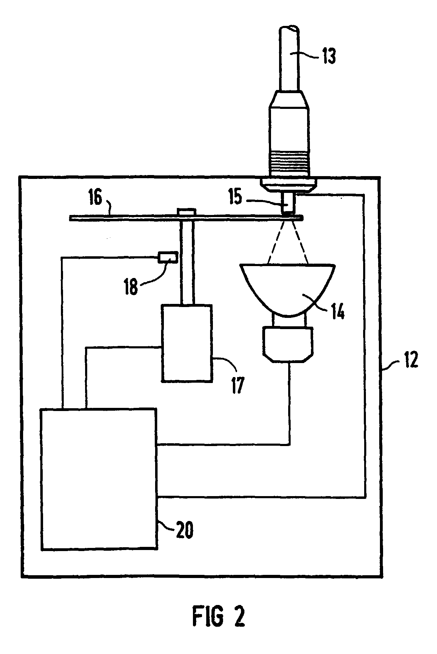

Eine vorteilhafte Version dieser Alternativlösung wird anhand der Fig. 2 näher beschrieben. Extern der Kamera 1 ist ein Lichtmodul 12 angeordnet, welches mittels Kabel 13 mit der Kamera 1 verbunden ist. Im Lichtmodul 12 befindet sich eine übliche Lichtquelle 14, die weißes Licht emittiert. Das Licht wird über eine im Kabel 13 verlegte Faseroptik 15 (Lichtleiter) zur Kameraoptik (Fig. 1) geleitet. (Die Lichtquelle 2 in Fig. 1 ist dann nicht vorhanden). Das Licht der drei Farben kann mittels einer im Lichtmodul 12 angeordneten Filterscheibe 16 erzeugt werden, die rotierend zwischen Lichtquelle 14 und Lichteintritt in die Faseroptik 15 angeordnet ist. Die Filterscheibe 16 kann aus drei Sektoren oder Segmenten bestehen, die für drei verschiedene Farben durchlässig sind und z.B. durch einen Schrittmotor 17 gedreht werden. Die Position der Scheibe kann durch einen Winkelgeber 18 erfaßt werden. Im Meßmodus steht die Scheibe auf einer Farbe still. Im Suchbild-Modus rotiert die Scheibe, so daß das Objekt nacheinander mit den drei Farben beleuchtet wird. Der CCD-Sensor 10 in der Kamera nimmt die Bilder nacheinander auf. In einer Bildverarbeitungselektronik 20, die auch die Motorsteuerung beinhalten kann, werden diese drei Bilder zu Videosignalen verarbeitet, die anschließend in einem sogenannten Framegrabber eingefroren werden können.

## Patentansprüche

1. Verfahren zur rechnergestützten Restauration von Zähnen, unter Verwendung einer 3D-Meßkamera mit der von dem aufzunehmenden Objekte ein monochromes 3D-Bild erzeugt wird, **dadurch gekennzeichnet,** daß zur besseren Bildinterpretation vom gleichen Objekt Farb-Videobilder erzeugt, auf einem Bildschirm dargestellt und mit dem monochromen 3D-Bild zur Überlagerung gebracht werden, wobei beide Aufnahmen mit ein und derselben Kamera gemacht werden.

2. Vorrichtung zur rechnergestützten Restauration von Zähnen unter Verwendung einer 3D-Meßkamera, mit der von dem aufzunehmenden Objekt einerseits ein monochromes 3D-Bild erzeugt wird, indem auf das gegebenenfalls oberflächenbeschichtete Meßobjekt ein Streifengitter projiziert, unter einem Parallaxewinkel aufgenommen und zu dem monochromen 3D-Bild weiterverarbeitet wird, **dadurch gekennzeichnet,** daß die 3D-Meßkamera auch zur Aufnahme von Farb-Videobildern und zu deren Überlagerung mit dem monochromen 3D-Bild ausgebildet ist, wobei eine ein weißes Licht emittierende Lichtquelle vorhanden ist und die 3D-Meßkamera (1) mit einem Farb-CCD (10') und mit einer vorgesetzten Farbfiltermaske versehen ist, und wobei die vorgesetzte Farbfiltermaske jeweils für die spezifische Farbe des Pixels und für die Farbe des Meßsystems durchlässig ist.

3. Vorrichtung zur rechnergestützten Restauration von Zähnen, mit einer 3D-Meßkamera, mit der von dem aufzunehmenden Objekt einerseits ein monochromes 3D-Bild erzeugt wird, indem auf das gegebenenfalls oberflächenbeschichtete Meßobjekt ein Streifengitter projiziert, unter einem Parallaxewinkel aufgenommen und zu dem monochromen 3D-Bild weiterverarbeitet wird, **dadurch gekennzeichnet,** daß die 3D-Meßkamera auch zur Aufnahme von Farb-Videobildern und zu deren Überlagerung mit dem monochromen 3D-Bild ausgebildet ist, wobei die 3D-Meßkamera einen beliebigen, vorzugsweise einen Schwarz-weiß-CCD-Sensor (10) enthält und Mittel (16) vorhanden sind, durch die das Objekt sequentiell mit wenigstens drei Farben beleuchtet oder das vom Objekt reflektierte Licht sequentiell in wenigstens drei Farben zerlegt werden kann.

4. Vorrichtung nach Anspruch 3, **dadurch gekennzeichnet,** daß als Lichtquelle wenigstens drei gepulste LED's vorgesehen sind.

5. Vorrichtung nach Anspruch 3, **dadurch gekennzeichnet,** daß eine weißes Licht emittierende Lichtquelle (14) vorhanden ist und in deren Beleuchtungs- oder Reflexionslicht-Strahlengang eine rotierende oder oszillierende Farbfilterscheibe (16) bringbar ist, die in getrennten Segmenten für zumindest drei verschiedene Farben durchlässig ausgebildet ist.

6. Vorrichtung nach einem der Ansprüche 2 bis 5, **dadurch gekennzeichnet,** daß die Lichtquelle (14) in einem extern der Kamera (1) angeordneten Lichtmodul (12) untergebracht ist.

7. Vorrichtung nach einem der vorhergehenden Ansprüche , **dadurch gekennzeichnet,** daß Mittel vorhanden sind, mit denen die in Beziehung zu setzenden 3D- und Video-Bilder in ihrer Größe und Projektionsrichtung angepaßt werden können.

8. Vorrichtung nach einem der Ansprüche 2 bis 7, **dadurch gekennzeichnet,** daß ein Rechner vorhanden ist, mit dem die Bildüberlagerung durchführbar ist.

## Claims

1. Process for computer-assisted restoration of teeth, using a 3D measuring camera with which a monochrome 3D image of the object to be recorded is generated, characterized in that, for better image interpretation of the same object, colour video images are generated, displayed on a screen and superimposed on the monochrome 3D image, both recordings being made with one and the same camera.

2. Device for computer-assisted restoration of teeth using a 3D measuring camera with which on the one hand a monochrome 3D image of the object to be recorded is generated, in that a grating is projected onto the possibly surface-coated object to be measured, is recorded at a parallax angle and is further processed to form the monochrome 3D image, characterized in that the 3D measuring camera is also designed for recording colour video images and for superimposing them on the monochrome 3D image, a light source emitting a white light being present and the 3D measuring camera (1) being provided with a colour CCD (10') and with an attached colour filter mask being respectively transparent to the specific colour of the pixel and to the colour of the measuring system.

3. Device for computer-assisted restoration of teeth, having a 3D measuring camera with which on the one hand a monochrome 3D image of the object to be recorded is generated, in that a grating is projected onto the possibly surface-coated object to be measured, is recorded at a parallax angle and is further processed to form the monochrome 3D image, characterized in that the 3D measuring camera is also designed for recording colour video images and for superimposing them on the monochrome 3D image, the 3D measuring camera including any desired, preferably a monochrome CCD sensor (10) and there being means (16) present by which the object can be sequentially illuminated with at least three colours or the light reflected from the object can be sequentially broken up into at least three colours.

4. Device according to Claim 3, characterized in that at least three pulsed LEDs are provided as the light source.

5. Device according to Claim 3, characterized in that a white-light-emitting light source (14) is provided and a rotating or oscillating colour filter disc (16), which is formed in separate segments transparent to at least three different colours, can be brought into the path of rays of the illuminating or reflection light of the said light source.

6. Device according to one of Claims 2 to 5, characterized in that the light source (14) is accommodated in a light module (12) arranged outside the camera (1).

7. Device according to one of the preceding claims, characterized in that there are means present by which the 3D and video images to be brought into a relationship can be adapted in their size and direction of projection.

8. Device according to one of Claims 2 to 7, characterized in that a computer is present by which the image superimposing can be carried out.

## Revendications

1. Procédé de restauration de dents, assistée par ordinateur, moyennant l'utilisation d'une caméra de mesure tridimensionnelle, avec laquelle une image monochrome tridimensionnelle de l'objet à enregistrer est produite, caractérisé en ce que pour une meilleure interprétation de l'image, on forme des images vidéo en couleurs du même objet, on les représente sur un écran et on les superpose à l'image monochrome tridimensionnelle, les deux enregistrements étant exécutés à l'aide d'une même caméra.

2. Dispositif pour la restauration de dents, assistée par ordinateur, moyennant l'utilisation d'une caméra de mesure tridimensionnelle, à l'aide de laquelle d'une part on forme une image monochrome tridimensionnelle de l'objet à enregistrer en projetant, sur l'objet de mesure dont la surface est éventuellement pourvue d'un revêtement, une grille formée de bandes, on effectue l'enregistrement sous un angle de parallaxe et on exécute ensuite un traitement pour obtenir l'image monochrome tridimensionnelle, caractérisé en ce que la caméra de mesure tridimensionnelle est également agencée pour l'enregistrement d'images vidéo en couleurs et pour leur superposition à l'image monochrome tridimensionnelle, auquel cas il est prévu une source de lumière émettant une lumière blanche et la caméra de mesure tridimensionnelle (1) est pourvue d'un dispositif CCD en couleurs (10') et d'un masque formant filtre coloré disposé en avant, et le masque formé d'un filtre coloré disposé en avant est transparent respectivement pour la couleur spécifique du pixel et pour la couleur du système de mesure.

3. Dispositif pour la restauration de dents, assistée par ordinateur, comportant une caméra de mesure tridimensionnelle, à l'aide de laquelle d'une part on forme une image monochrome tridimensionnelle de l'objet à enregistrer en projetant, sur l'objet de mesure dont la surface est éventuellement pourvue d'un revêtement, une grille formée de bandes, on effectue l'enregistrement sous un angle de parallaxe et on exécute ensuite un traitement pour obtenir l'image monochrome tridimensionnelle, caractérisé en ce que la caméra de mesure tridimensionnelle est également agencée pour l'enregistrement d'images vidéo en couleurs et pour leur superposition avec l'image monochrome tridimensionnelle, et dans lequel la caméra de mesure tridimensionnelle contient un capteur quelconque, de préférence un capteur CCD en noir et blanc (10) et il est prévu des moyens (16) grâce auxquels l'objet est éclairé séquentiellement avec au moins trois couleurs, ou la lumière réfléchie par l'objet peut être subdivisée séquentiellement en au moins trois couleurs.

4. Dispositif selon la revendication 3, caractérisé en ce qu'au moins trois diodes LED commandées de manière pulsée sont prévues en tant que source de lumière.

5. Dispositif selon la revendication 3, caractérisé en ce qu'il est prévu une source de lumière (14) émettant une lumière blanche, et un disque à filtre coloré (16) rotatif ou oscillant, qui est agencé de manière à être transparent, dans des segments séparés, pour au moins trois couleurs différentes, peut être amené dans le trajet du faisceau de la lumière d'éclairement ou de la lumière réfléchie de cette source.

6. Dispositif selon l'une des revendications 2 à 5, caractérisé en ce que la source de lumière (14) est logée dans un module (12) délivrant une lumière, qui est disposé à l'extérieur de la caméra (1).

7. Dispositif selon l'une des revendications précédentes, caractérisé en ce qu'il est prévu des moyens à l'aide desquels on peut adapter la grandeur et la direction de projection des images tridimensionnelles et des images vidéo, qui doivent être mises réciproquement en rapport.

8. Dispositif selon l'une des revendications 2 à 7, caractérisé en ce qu'il est prévu un calculateur à l'aide duquel la superposition des images peut être exécutée.
